# EUROPEAN PATENT SPECIFICATION

(11) **EP 1 916 016 B1**
(45) Date of publication and mention of the grant of the patent: **22.03.2017**
(21) Application number: 07116614.4
(22) Date of filing: 18.09.2007
(51) Int. Cl.: A61N 5/06

(54) **Covering for treating the skin and dermis**
Abdeckung zur Behandlung der Haut sowie der Dermis
Protection pour le traitement de la peau et du derme

(30) Priority: 24.10.2006 IT BO20060735
(43) Date of publication of application: 30.04.2008
(73) Proprietor: Pomar, Rodolfo, 40067 Pianoro, Frazione Rastignano (BO) (IT)
(72) Inventor: Pomar, Rodolfo, 40067 Pianoro, Frazione Rastignano (BO) (IT)
(74) Representative: Manzella & Associati

(56) References cited:
- US-A1- 2003 167 080
- US-A1- 2006 217 690
- US-B1- 6 471 716
- US-B1- 6 596 016

## Description

The present invention relates to a covering for treating the skin and dermis for aesthetic and/or therapeutic purposes: in particular, it may be a mask for the face or for other parts of the body or a particular type of band which can be superimposed on portions of the skin of the user.

It is known that by subjecting the dermis of a person to appropriate cycles of lighting with light beams of preset wavelengths it is possible to treat certain blemishes and contrast certain problems of the skin itself.

The design choice that offers the greatest advantages in treating the skin provides for the use of small assemblies (provided with a light source) which can be directed toward a small portion of the dermis and arranged at short distances therefrom.

Among these, the embodiment disclosed in EPA 04025840.2 of October 29, 2004 and entitled "Light irradiation unit for diagnosing and treating skin problems" offers the extremely advantageous possibility to substantially replace the source (or a filter interposed between the source and the skin), using, for each type of skin, the light source that is most suitable in combination with an appropriate treatment cycle.

The diffusion of light-emitting diodes and the corresponding extension of their possible fields of application has recently led some manufacturers to study devices for treating the skin and dermis which use these diodes as light sources.

US 2006/0217690 discloses that the array of diodes may comprise a formable sheet that can be manipulated to meet the curvature of the body area to be treated, but not by using a calculation of the distance.

Among the advantages provided by diodes with respect to other sources, in addition to the fact that they have the first failures linked to aging after enormously longer times than other light sources, there is the extreme precision of the wavelength of the emitted beam: this entails first of all the possibility to avoid using filters to eliminate dangerous frequencies and secondly an extreme specificity of each treatment.

This type of device is constituted by a basic supporting structure, with respect to which a sort of screen can move and is orientable, its surface comprising a distributed plurality of light-emitting diodes.

Treatment is performed by moving the screen close to the face (or to the part to be treated) and starting a cycle of emissions.

The surfaces of the face (in particular) and of the other parts of the body which can benefit from a treatment of this kind, however, are extremely irregular. Consider in particular the face: the nose protrudes, some regions are in shadow when others are perfectly illuminated, and therefore it is normal for the treatment to be rather uneven.

Every known type of apparatus is adapted to irradiate the treated region with a light beam of a certain wavelength, which depends on the characteristics of the installed diodes: to perform different treatments, it is necessary to have available several different appliances, each of which emits a stream of a specific wavelength.

A severe drawback which arises from the possibility to use several devices having different characteristics, in addition to space occupation and installation, is linked to cost, which for devices of this type is extremely high and makes it almost prohibitive to have an apparatus available for each wavelength of interest.

Document US 2003/0167080 A1 teaches, for example different arrangements of plural leds, that are embedded in an elastic material, and are connected to unit that modulates their light frequency and wavelengths.

The aim of the present invention is to obviate the above mentioned drawbacks and meet the mentioned requirements, by providing a covering for treating the skin and dermis with light beams emitted by light-emitting diodes, which is compact and suitable for the uniform treatment of delimited areas.

Within this aim, an object of the present invention is to provide a series of interchangeable coverings, each of which emits, by means of diodes, a light beam of a certain wavelength, said coverings being adapted to be installed in an appropriate unit for treating the skin and dermis.

Another object of the present invention is to provide a covering which is simple, relatively easy to provide in practice, safe in use, effective in operation, and has a relatively low cost.

This aim and these and other objects which will become better apparent hereinafter by the present covering and its use for treating the skin and dermis of a user according to the present invention, that has the features set forth in claims 1 and 6.

Further characteristics and advantages of the invention will become better apparent from the following detailed description of a preferred but not exclusive embodiment of a covering for treating the skin and dermis, illustrated by way of non-limiting example in the accompanying drawings, wherein:

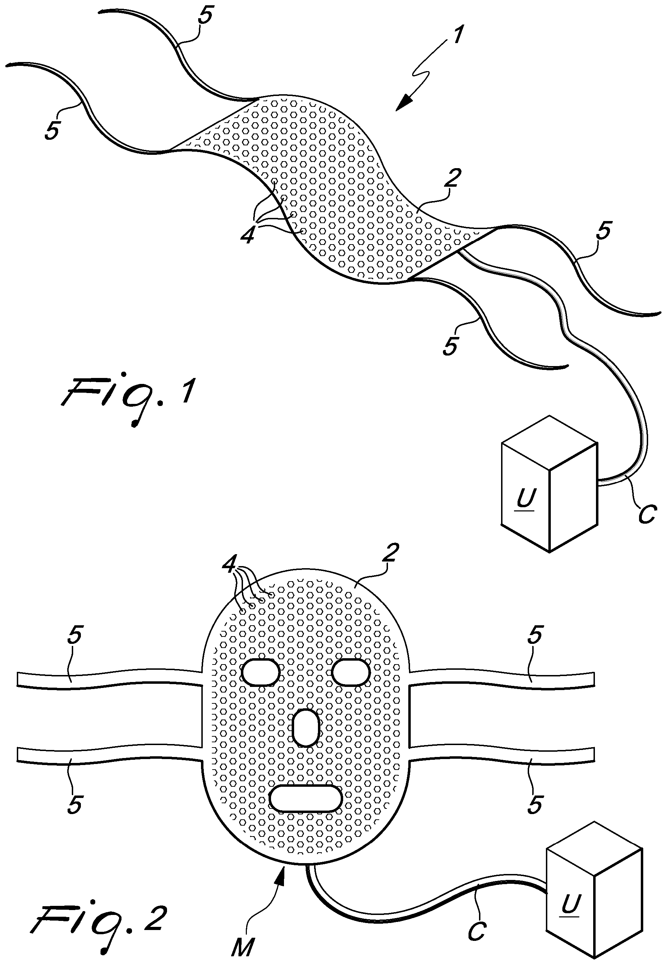

Figure 1 is a perspective view of a unit provided with a first embodiment of a covering according to the invention;
Figure 2 is a perspective view of a unit provided with a second embodiment of a covering according to the invention;

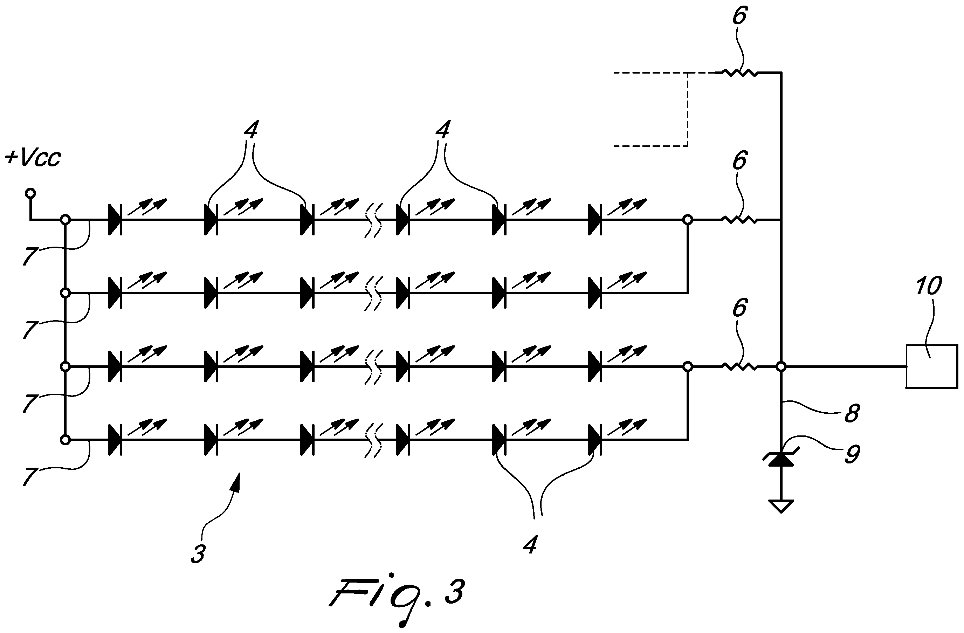

Figure 3 is a circuit diagram of an embodiment of a covering according to the invention.

With reference to the figures, the reference numeral 1 generally designates a covering for treating the skin and dermis.

The covering 1 comprises a laminar body 2, which contains electrical and electronic circuits 3 for management and control of light sources 4: according to the embodiments shown, the light sources are constituted by a distributed plurality of light-emitting diodes 4 (hereinafter referenced simply by the acronym LED, Light Emitting Diode). Of course, it is also possible to consider providing a covering which has a single LED 4 and is intended for the point-like treatment of a particular portion of the skin of the patient.

The laminar body 2 can be made substantially of various materials and in various shapes: any rigidity of the body 2 can ensure a certain structural capability, which is adapted to ensure optimum treatment of certain parts of the dermis; in other cases, its maximum flexibility ensures perfect adhesion even to irregular and curved surfaces of the dermis.

The LEDs 4 are embedded within the laminar body 2, which for this purpose has a thickness which is suitable to accommodate said components and the corresponding circuits 3.

The circuit 3 is connected to a control and management unit U by means of a suitable cable C.

In order to optimize use and make the covering 1 adapted to be used on any part of the body of the patient, wrapping around it uniformly, the laminar body 2 has limited consistency and stiffness: it is possible to use various materials to provide the body 2, for example leathers and/or hide, polymeric materials (either woven or obtained by molding, lamination, et cetera), but also fabric and/or derivatives of paper.

The body 2 further has suitable retention elements 5: the elements 5 may be constituted preferably by ribbons (or straps), the free ends of which can be mutually coupled (either simply by tying them or by using buckles and engagement elements).

According to an embodiment of particular practical interest, the surface of the LED diodes 4 directed toward the portion of the skin/dermis to be treated can be covered by means of a suitable filter (not shown in the figure): such filter is adapted to eliminate any dangerous frequencies that might be present in the emission spectrum of the LED 4 that is associated therewith.

In order to homogenize the treatment of the skin of the patient, the LEDs 4 are all arranged at the same distance from the surface of the skin to be treated, a distance which is calculated as a function of the characteristics of the intensity and frequency of the beam emitted by the particular type of LED 4.

Depending on the treatment to be performed, it is possible to provide dedicated coverings 1, equipped with LEDs 4 which have different characteristics; for example, it is possible to have LEDs 4 adapted to emit a red beam of light to stimulate the production of collagen, or it is possible to have LEDs 4 which are adapted to emit a blue beam of light in order to contrast bacterial acne, or, finally, arrange diodes 4 adapted to emit a yellow beam of light to stimulate the lymphatic system and the nervous system. Of course, it is also possible to combine the presence of LEDs 4 having different characteristics in a same covering 1 in order to make it adapted to perform mixed treatments.

According to an embodiment of particular practical interest, the laminar body 2 can be shaped substantially like a mask M, or like a supporting surface.

This configuration is of course ideal for treatments of the face of the patient: the mask M can in fact be arranged on the face of the patient to be treated and be rigidly coupled to the head by means of the retention elements 5.

Depending on the requirements of the treatment, the mask M can have or not openings at the eyes, nose and mouth of the patient: in certain cases, it might be useful to treat the eyelids and/or lips and therefore such openings would be counterproductive.

The LEDs 4 are correctly polarized by means of resistors 6 which are arranged in series thereto.

Further, the LEDs 4, according to a possible circuit embodiment, are arranged in series on respective branches 7.

In turn, the branches 7 are arranged in parallel to each other with respect to the high voltage level and the control signal, a branch 8 being derived toward the ground, before the respective connection to the control signal, on which a Zener diode 9 is connected. The control signal reaches the LEDs 4 by means of a connector 10 to which the cable C is connected.

The operation of the invention is as follows: by arranging the covering 1 suitable for the treatment that the user is to undergo, it is possible to benefit from a controlled beam of light which is emitted by the LEDs 4 and interacts with the dermis, improving its appearance and facilitating the healing of certain disorders.

Positively, the surface to be treated is struck by a uniform light beam, which arrives from sources which are all equidistant from the skin, providing an optimum treatment even on small portions of the skin or on regions which are normally in shadow.

Advantageously, a same unit U can operate with different coverings 1, for example having LEDs 4 with emissions of different colors.

It has thus been shown that the invention achieves the intended aim and objects.

The invention thus conceived is susceptible of numerous modifications and variations, all of which are within the scope of the appended claims.

All the details may further be replaced with other technically equivalent ones.

In the exemplary embodiments shown, individual characteristics, given in relation to specific examples, may actually be interchanged with other different characteristics that exist in other exemplary embodiments.

Moreover, it is noted that anything found to be already known during the patenting process is understood not to be claimed and to be the subject of a disclaimer.

In practice, the materials used, as well as the shapes and dimensions, may be any according to requirements without thereby abandoning the scope of the protection of the appended claims.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. Use of a covering for non-therapeutic cosmetic treating of the skin and dermis of a user, wherein the covering that comprises a laminar body (2) of molded polymeric material is fixed, in use, to the area to be treated of a body of the user by means of suitable retention elements (5); at least one light source (4) is connected to circuits (3) for power supply and control thereof, the light source and the circuits being arranged and mounted on said laminar body (2), said light source is constituted by a plurality of light emitting diodes (4) suitable to emit a beam of light of red color or blue color or yellow color, that are distributed on the internal surface of, and embedded within said laminar body (2) that has a thickness suitable to accommodate therein also said circuits (3), the laminar body (2) contains said plurality of diodes (4) and said circuits (3) are connected to a control and management unit (U) by means of an appropriate cable (C), **characterized in that** with the laminar body (2) fixed in use to the area of the body of the user to be treated by means of said retention means (5) said plurality of diodes (4) embedded in said laminar body (2) are all arranged equidistant from the surface of the skin of the user to be treated in order to homogenize the treatment and emit a uniform beam of light, the distance from the surface of the skin to be treated being established by calculation as a function of the characteristics of the intensity and frequency of the beam emitted by the particular type of diodes (4) embedded in said laminar body (2).

2. The use according to claim 1, **characterized in that** the surface of said diodes (4) which is directed toward the portion of the skin to be treated is covered by means of a suitable optical filter.

3. The use according to claims 1 or 2, **characterized in that** said laminar body (2) is shaped substantially like a mask (M) that is arranged in use on the face of the patient to be treated and rigidly coupled to the head by means of said retention elements (5), which are constituted generally by ribbons which can be mutually associated at their ends.

4. The use according to one or more of the preceding claims, **characterized in that** said diodes (4) are correctly polarized by means of resistors (6) which are arranged in series thereto.

5. The use according to one or more of the preceding claims, **characterized in that** said diodes (4) are arranged in series on respective branches (7), which are in parallel to each other with respect to the high voltage level and the control signal, a branch (8) being derived toward the ground before the respective connection to the control signal, on which a Zener diode (9) is connected.

6. A covering for non-therapeutic cosmetic treating of the skin and dermis of a user, comprising a laminar body (2) of molded polymeric material fixed, in use, to the area to be treated of a body of the user by means of suitable retention elements (5); at least one light source (4) connected to circuits (3) for power supply and control thereof, the light source and the circuits being arranged and mounted on said laminar body (2), said light source being constituted by a plurality of light emitting diodes (4) suitable to emit a beam of light of red color or blue color or yellow color, that are distributed on the internal surface of, and embedded within said laminar body (2) that has a thickness suitable to accommodate therein also said circuits (3), the laminar body (2) containing said plurality of diodes (4) and said circuits (3) being connected to a control and management unit (U) by means of an appropriate cable (C), **characterized in that** wherein with the laminar body (2) fixed in use to the area of the body of the user to be treated by means of said retention means (5) said plurality of diodes (4) embedded in said laminar body (2) are all arranged equidistant from the surface of the skin of the user to be treated in order to homogenize the treatment and emit a uniform beam of light, the distance from the surface of the skin to be treated being established by calculation as a function of the characteristics of the intensity and frequency of the beam emitted by the particular type of diodes (4) embedded in said laminar body (2).

## Patentansprüche

1. Verwendung einer Abdeckung für die nicht therapeutische kosmetische Behandlung der Haut und Dermis eines Verwenders, wobei die Abdeckung, die einen Laminarkörper (2) aus modelliertem Polymermaterial umfasst, beim Gebrauch mittels geeigneter Halteelemente (5) an dem Körperbereich des Verwenders befestigt ist, der behandelt werden muss; mindestens eine Lichtquelle (4) ist mit Stromversorgungs- und Kontrollschaltkreisen (3) derselben verbunden, wobei die Lichtquelle und die Schaltkreise auf dem genannten Laminarkörper (2) angeordnet und montiert sind, die genannte Lichtquelle besteht aus einer Mehrzahl von Photoemitterdioden (4), die dazu geeignet sind, einen roten oder blauen oder gelben Lichtstrahl auszustrahlen, auf der inneren Oberfläche des genannten Laminarkörpers (2) verteilt und in ihn eingebettet sind, der eine Stärke hat, die dazu geeignet ist, in ihm auch die genannten Schaltkreise (3) aufzunehmen, wobei der Laminarkörper (2) die genannte Mehrzahl von Dioden (4) enthält und die genannten Schaltkreise (3) mit einem entsprechenden Kabel (C) mit einer Kontroll- und Steuerungseinheit (U) verbunden sind, **dadurch gekennzeichnet, dass** die genannte Mehrzahl von Dioden (4), die in den genannten Laminarkörper (2) eingebettet ist, mit beim Gebrauch mittels der genannten Halteelemente (5) an dem zu behandelnden Körperbereich des Verwenders befestigten Laminarkörper (2), alle in gleichem Abstand von der zu behandelnden Hautoberfläche des Verwenders angeordnet sind, um die Behandlung zu vereinheitlichen und einen gleichmäßigen Lichtstrahl auszustrahlen, wobei der Abstand von der zu behandelnden Hautoberfläche in Abhängigkeit von den Stärke- und Frequenzeigenschaften des Lichtstrahls berechnet wird, der von dem besonderen Typ von Dioden (4) ausgestrahlt wird, die in den genannten Laminarkörper (2) eingebettet sind.

2. Verwendung nach Anspruch 1, **dadurch gekennzeichnet, dass** die Oberfläche der genannten Dioden (4), die auf den zu behandelnden Hautteil gerichtet ist, mittels eines entsprechenden optischen Filters verkleidet ist.

3. Verwendung nach den Ansprüchen 1 und 2, **dadurch gekennzeichnet, dass** der genannte Laminarkörper (2) im wesentlichen die Form einer Maske (M) hat, die beim Gebrauch auf das Gesicht des zu behandelnden Patienten gelegt und mit den genannten Halteelementen (5), die im Allgemeinen aus Bändern bestehen, die sich an ihren Enden miteinander verbinden lassen, am Kopf befestigt wird.

4. Verwendung nach einem oder mehreren der vorausgehenden Ansprüche, **dadurch gekennzeichnet, dass** die genannten Dioden (4) durch Widerstände (6) korrekt polarisiert sind, die dazu in Reihe angeordnet sind.

5. Verwendung nach einem oder mehreren der vorausgehenden Ansprüche, **dadurch gekennzeichnet, dass** die genannten Dioden (4) in Reihe auf den jeweiligen Zweigen (7) angeordnet sind, die untereinander parallel im Verhältnis zum hohen Spannungsniveau und dem Steuersignal angeordnet sind, wobei ein Zweig (8) nach Masse abgeleitet ist und dies vor der jeweiligen Verbindung mit dem Kontrollsignal, an das eine Zenerdiode (9) angeschlossen ist.

6. Abdeckung für die nicht therapeutische kosmetische Behandlung der Haut und Dermis eines Verwenders, die einen Laminarkörper (2) aus modelliertem Polymermaterial umfasst und beim Gebrauch durch geeignete Halteelemente (5) am zu behandelnden Körperbereich des Verwenders befestigt ist; mindestens eine Lichtquelle (4), die mit Stromversorgungs- und Kontrollschaltkreisen (3) verbunden ist, wobei die Lichtquelle und die Schaltkreise auf dem genannten Laminarkörper (2) angeordnet und montiert sind und die genannte Lichtquelle aus einer Mehrzahl von Photoemitterdioden (4) besteht, die dazu geeignet sind, einen roten oder blauen oder gelben Lichtstrahl auszustrahlen, auf der inneren Oberfläche des genannten Laminarkörpers (2) verteilt und in ihn eingebettet sind, der eine Stärke hat, die dazu geeignet ist, in seinem Inneren auch die genannten Schaltkreise (3) aufzunehmen, wobei der Laminarkörper (2) die genannte Mehrzahl von Dioden (4) enthält und die genannten Schaltkreise (3) mit einem entsprechenden Kabel (C) mit einer Kontroll- und Steuerungseinheit (U) verbunden sind, **dadurch gekennzeichnet, dass** die genannte Mehrzahl von Dioden (4), die in den genannten Laminarkörper (2) eingebettet ist, mit beim Gebrauch durch die genannten Halteelemente (5) an dem zu behandelnden Körperbereich des Verwenders befestigten Laminarkörper (2), in gleichem Abstand von der zu behandelnden Hautoberfläche des Verwenders angeordnet sind, so dass sie die Behandlung vereinheitlichen und einen gleichmäßigen Lichtstrahl ausstrahlen, wobei der Abstand von der zu behandelnden Hautoberfläche in Abhängigkeit von den Stärke- und Frequenzeigenschaften des Lichtstrahls berechnet wird, der von dem besonderen Typ von Dioden (4) ausgestrahlt wird, die in den genannten Laminarkörper (2) eingebettet sind.

## Revendications

1. Utilisation d'une calotte pour le traitement cosmétique non thérapeutique de la peau et du derme d'un utilisateur, où la calotte, qui comprend un corps laminaire (2) de matériau polymérique modelé est fixée, pendant l'utilisation, à la zone du corps de l'utilisateur qui doit être traitée au moyen d'éléments de contrainte (5) adéquats ; au moins une source (4) de lumière est connectée aux circuits (3) d'alimentation électrique et de contrôle de celle-ci, étant la source de lumière et les circuits disposés et montés sur ledit corps laminaire (2), ladite source de lumière est constituée d'une pluralité de diodes photoémettrices (4) aptes à émettre un faisceau lumineux de couleur rouge ou bleu ou jaune, distribuées sur la surface intérieure de, et serties dans ledit corps laminaire (2), lequel a une épaisseur apte à accueillir en lui-même aussi lesdits circuits (3), le corps laminaire (2) contient ladite pluralité de diodes (4) et lesdits circuits (3) sont connectés à une unité (U) de contrôle et gestion à travers un câble (C) prévu à cet effet, **caractérisée en ce qu'**avec le corps laminaire (2) fixé pendant l'utilisation à la partie du corps de l'utilisateur à traiter au moyen desdits éléments de contrainte (5) ladite pluralité de diodes (4) sertie dans ledit corps laminaire (2) sont toutes disposées de manière équidistante par rapport à la surface de la peau de l'utilisateur à traiter afin de rendre homogène le traitement et émettre un faisceau lumineux uniforme, étant la distance de la surface de la peau à traiter calculée en fonction des caractéristiques d'intensité et de fréquence du faisceau émis par le type particulier de diodes (4) incorporées dans ledit corps laminaire (2).

2. Utilisation selon la revendication 1, **caractérisée en ce que** la surface desdites diodes (4) qui est dirigée vers la partie de peau à traiter est revêtue au moyen d'un filtre optique spécifique.

3. Utilisation selon les revendications 1 et 2, **caractérisée en ce que** ledit corps laminaire (2) est sensiblement en forme de masque (M) qui pendant l'utilisation est placé sur le visage du patient à traiter et lié à la tête au moyen desdits éléments de contrainte (5) qui sont généralement constitués de rubans réciproquement associables à leurs extrémités.

4. Utilisation selon une ou plusieurs des revendications précédentes, **caractérisée en ce que** lesdites diodes (4) sont correctement polarisées par le biais de résistances (6) disposées en série entre elles.

5. Utilisation selon une ou plusieurs des revendications précédentes, **caractérisée en ce que** lesdites diodes (4) sont disposées en série sur des sections respectives (7), qui sont disposées en parallèle entre elles par rapport au niveau haut de tension et le signal de commande, une section (8) étant dérivée vers la masse, avant le branchement respectif au signal de contrôle, sur lequel est branchée une diode Zener (9).

6. Calotte pour le traitement cosmétique non thérapeutique de la peau et du derme d'un utilisateur, comprenant un corps laminaire (2) de matériau polymérique modelé et fixé, pendant l'utilisation, à la zone du corps de l'utilisateur à traiter par le biais d'éléments de contrainte (5) adéquats ; au moins une source (4) de lumière connectée à des circuits (3) pour l'alimentation électrique et le contrôle, étant la source de lumière et les circuits disposés et montés sur ledit corps laminaire (2), étant ladite source de lumière constituée d'une pluralité de diodes (4) photoémettrices aptes à émettre un faisceau lumineux de couleur rouge ou bleu ou jaune, distribuées sur la surface intérieure de, et serties dans ledit corps laminaire (2), lequel a une épaisseur apte à accueillir en lui-même aussi lesdits circuits (3), le corps laminaire (2) contenant ladite pluralité de diodes (4) et lesdits circuits (3) étant connectés à une unité (U) de contrôle et gestion par le biais d'un câble (C) adéquat, **caractérisé en ce qu'**avec le corps laminaire (2) fixé pendant l'utilisation à la zone du corps de l'utilisateur à traiter par le biais desdits éléments de contrainte (5) ladite pluralité de diodes (4) sertie dans ledit corps laminaire (2) sont disposées de manière équidistante par rapport à la surface de la peau de l'utilisateur à traiter afin de rendre homogène le traitement et émettre un faisceau lumineux uniforme, étant la distance de la surface de la peau à traiter établie par calcul en fonction des caractéristiques d'intensité et de fréquence du faisceau émis par le type particulier de diodes (4) incorporées dans ledit corps laminaire (2).
